# EUROPEAN PATENT APPLICATION

(11) **EP 0 985 410 A1**
(43) Date of publication of application: **15.03.2000**
(21) Application number: 99402197.0
(22) Date of filing: 07.09.1999
(51) Int. Cl.: A61K 7/48, A61K 7/06

(54) **External application composition containing a carboxyvinyl polymer and gellan gum**

(30) Priority: 08.09.1998 JP 27259098
(71) Applicant: SHISEIDO COMPANY LIMITED, Chuo-ku Tokyo (JP)
(72) Inventor: Abe, Koji, c/o Shiseido Research Center (1), Yokohama-shi, Kanagawa 223-8553 (JP); Miyahara, Reiji, c/o Shiseido Research Center (1), Yokohama-shi, Kanagawa 223-8553 (JP); Sakai, Tetsuya, c/o Shiseido Research Center (1), Yokohama-shi, Kanagawa 223-8553 (JP); Nanba, Tomiyuki, c/o Shiseido Research Center (1), Yokohama-shi, Kanagawa 223-8553 (JP)
(74) Representative: Rinuy, Santarelli

(57) **Abstract**

An external application composition comprising:
(A) at least one carboxyvinyl polymer selected from the group consisting of carboxyvinyl polymers and alkylmodified carboxyvinyl polymers; and
(B) gellan gum.

## Description

### BACKGROUND OF THE INVENTION

### 1. Field of the Invention

The present invention relates to an external application composition. More specifically, it relates to an external application composition having the improved stability and the improved feelings at use, while maintaining the viscosity of the external application composition.

### 2. Description of the Related Art

The important factor for evaluating the external application composition typically represented by cosmetic compositions are the stability and the feelings at use. The typical known means for improving, especially, the stability is the formulation of a substantial amount of a surfactant into the composition.

However, the external application composition containing a substantial amount of a surfactant has a problem of the feelings at use in many cases. In addition, in view of the further consideration of the safety and environmental protection in the recent years, the amount of the surfactant formulated in the external application is required to be decreased.

Accordingly, attempts have been made to formulate water-soluble polymers such as carboxyvinyl polymers and alkyl-modified carboxyvinyl polymers and polymeric polysaccharides such as gellan gum to improve the feelings at use and the stability.

However, there are problems in the formulation of carboxyvinyl polymers and alkyl-modified carboxyvinyl polymers that, since the carboxyvinyl polymers and the alkyl-modified carboxyvinyl polymers have poor salt resistance, they are not preferable to be formulated in the external application composition containing a salt. In the case of gellan gum, the especially unique useability can be provided with the incorporation thereof into the external application composition, but there was problem that the stability of the external application composition is difficult to maintain when gellan gum is formulated alone. Further, as disclosed in JP-A-9-20649, although the gellan gum is gelled in the presence of a cation, the gel becomes in the state similar to agar-agar. Thus, the resultant composition may be used in the field of, for example, foods, but the composition is inconvenient to use in the fields of, for example, cosmetics when the gellan gum is formulated into the composition.

### SUMMARY OF THE INVENTION

Accordingly, the object of the present invention is to improve the stable formulation of the gellan gum into the external application composition such as a cosmetic composition and to improve the salt resistance, while revealing the unique useability or feelings at use of the gellan gum.

Other objects and advantages of the present invention will be apparent from the following description.

In accordance with the present invention, there is provided an external application composition (i.e., "the present external application composition") comprising:
(A) at least one carboxyvinyl polymer, selected from the group consisting of carboxyvinyl polymers and alkyl-modified carboxyvinyl polymers; and
(B) gellan gum.

The term "external application composition" as used herein is intended to widely mean any composition capable of applying to a variety of an external skin, more specifically cosmetics, drugs, quasi-drugs, irrespective of the specifical products under the control of the pharmaceutical affairs law.

### DESCRIPTION OF THE PREFERRED EMBODIMENTS

The present inventors engaged in intensive study to solve the above-mentioned problems and, as a result, found that, in the external application composition having a combination of (A) the carboxyvinyl polymer and/or the alkoxy-modified carboxyvinyl polymer and (B) the gellan gum formulated therein, the stable external application composition having the increased viscosity of the overall composition and the extremely improved feelings at use can be unexpectedly obtained, without formulating a surfactant into the composition for the purpose of stabilizing the composition, by the combined function of the formulated components (A) and (B), even in the presence or absence of a salt, whereby the present invention has been accomplished.

The mode of the operation of the present invention will now be explained.

The gellan gum formulated into the present external application composition was developed by Kelco. Co., Ltd., U.S.A. and commercially available from Dainippon Pharmaceutical Co., Ltd. and contains, as a main constituent, heteropolysaccharides comprising glucose, glucuronic acid and rhamnose (glucose: glucuronic acid: rhamnose = 2:1:1), produced by Pseudomonas Elodea in a culture medium containing, as a main component, glucose. The gellan gum has a molecular weight thereof is about 670,000 - 920,000 and is an industrial product having a high purity, industrially produced under a complete production control. As shown in the following structural formula (I), the gellan gum is a linear polymeric polysaccharide comprising a repeating unit of 1,3-bonded glucose, 1,4-bonded glucuronic acid, 1,4-bonded glucose and 1,4-bonded rhamnose.

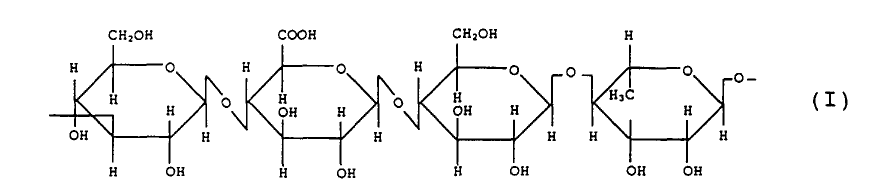

The formulation amount of the gellan gum in the present external application composition is preferably 0.01 to 1.5% by weight, especially preferably 0.02 - 1.0% by weight, based upon the total weight of the composition. When the amount is less than 0.01% by weight, the intended sufficient increase in the viscosity of the entire composition tends to unpreferably become substantially difficult. Contrary to this, when the amount is more than 1.5% by weight, the film specific to the polymer tends to form in the system, whereby the good feelings at use tend to be unpreferably impaired. In addition, when the gellan gum is formulated in an amount of more than said concentration in the composition, the entire composition tends to become in the state similar to agar-agar, which tends to remarkably make the composition unpreferable properties as the external application composition.

The carboxyvinyl polymer formulated, together with the gellan gum, into the present external application composition is an acidic polymer containing, as a main component, acrylic acid polymer and the commercially available products such as, for example, Hivis Wako 103, 104 and 105 (available from Wako Junyaku Kogyo K.K., Japan), Carbopol series (available from B.F. Goodrich Co., Ltd.), Aqupec series (available from Sumitomo Seika K.K., Japan), Junlon PW series (available from Nihon Junyaku Co., Ltd., Japan) can be used.

The alkyl-modified carboxyvinyl polymer formulated, together with gellan gum, into the present external application composition is a polymer mainly composed of alkyl acrylate methacrylate copolymer and may be a commercial product. Examples of such products are Carbopol 1342, Pemulen TR-1, Pemulen TR-2 (available from BF Goodrich Co., Ltd.), etc.

In the present external application composition, the gellan gum is used, in combination of either one of the above-mentioned carboxyvinyl polymer and alkyl-modified carboxyvinyl polymer, or both thereof. The amount of the carboxyvinyl polymer and/or alkyl-modified carboxyvinyl polymer is preferably 0.01 - 3.0% by weight, more preferably 0.1 - 1.0% by weight, based upon the total amount of the composition.

When this formulation amount is less than 0.01% by weight, the desired increase in the viscosity of the composition due to the interaction with gellan gum tends to unpreferably becomes difficult. Contrary to this, when the formulation amount is more than 3.0% by weight, the further improvement of the intended effect does not meet the increase in the formulation amount and the sufficient accomplishment of the intended effect of the present invention tends to unpreferably become difficult.

Furthermore, the formulation ratio of (B) the gellan gum to (A) the carboxyvinyl polymer and/or the alkyl-modified carboxyvinyl polymer is preferably 1:100 - 150:1, more preferably 1:50 - 20:1, in a weight ratio, depending upon the type of the product form of the present external application composition, and taking into consideration of the above-mentioned the formulation amount. When this formulation ratio is the outside of the above-mentioned preferable range by the use of the relatively large amount of the carboxyvinyl polymer and/or the alkyl-modified carboxyvinyl polymer, the carboxyvinyl polymer and/or the alkyl-modified carboxyvinyl polymer are wastefully consumed and also the sufficient accomplishment of the intended effects of the present invention tends to unpreferably become difficult. Contrary to this, when the amount of the gellan gum is relatively large, the film specific to a polymer tends to form in the composition, and therefore, the feelings at use tend to decrease and the entire composition tends to become in the state similar to that of agar-agar, which is inconvenient as an external application composition.

As explained, by the formulation of the gellan gum, in combination with the carboxyvinyl polymer and/or the alkyl-modified carboxyvinyl polymer, into, for example, a cosmetic composition, the stable external application composition with retaining a high viscosity, and having an extremely improved feelings at use, irrespective of the presence or absence of a salt, can be obtained.

The external application composition according to the present invention may optionally contain, in addition to the above essential constituent, other known components for drug compositions, as long as the desired effects of the present invention are not impaired.

Examples of such components are hydrocarbons such as liquid paraffin, squalane, vaseline; oil and fats such as Macadamia nut oil, olive oil, lanolin, jojova oil; waxes such as carnauba wax, candelilla wax; silicones such as dimethyl polysiloxane, methylphenyl siloxane; higher alcohols such as capryl alcohol, lauryl alcohol, myristyl alcohol, cetyl alcohol, cholesterol, phytosterol; higher fatty acids such as capric acid, myristic acid, palmitic acid, stearic acid, behenic acid, lanolin fatty acid, linoleic acid, linolenic acid; humectants such as polyethylene glycol, propylene glycol, dipropylene glycol, glycerol, 1,3-butylene glycol, erythritol, sorbitol, xylitol, multitol, mucopolysaccharides, hyaluronic acid, chondroitin sulfuric acid, chitin, chitosan; lower alcohols such as ethanol; antioxidants such as butylhydroxy toluene, tocopherol, ficin; antibiotic agent such as benzoic acid, salicyclic acid, sorbic acid, p-oxybenzoic acid alkyl esters, hexachlorophene.

Further examples of such components are amino acids such as glycine, alanine, valine, leucine, threonine, phenylalanine, tyrosine, aspartic acid, asparagine, glutamine, taurine, arginine, histidine, and alkali metal salts and hydrochlorides thereof; organic acids such as acyl sarcosinate (e.g., sodium N-lauryl sarcosinate), glutathione, citric acid, malic acid, tartaric acid, lactic acid; vitamin A and the derivatives thereof; vitamin Bs such as vitamin B6 hydrochloride, vitamin B6 tripalmitate, vitamin B6 dioctanoate, vitamin B2 and the derivatives thereof, vitamin B12, vitamin B15 and the derivatives thereof; vitamin Cs such as ascorbic acid, ascorbic acid sulfate esters (salts), ascorbic acid dipalmitate; vitamin Es such as α-tocopherol, β-tocopherol, γ-tocopherol, vitamin E acetate; vitamin Ds; other vitamines such as vitamin H, pantothenic acid, pantethine; various drugs or agents such as nicotinic amide, nicotinic benzyl, γ-oryzanol, allantoin, glycyrrhizic acid (salt), glycyrrhetic acid and the derivatives thereof, hinokitiol, bisabold, eucalyptone, thymol, inositol, saponins such as bupleurum root saponin, ginseng saponin, luffa cylindrica saponin, pantothenyl ethyl ether, ethinylestradiol, tranexamic acid, arbutin, cepharanthine, placental extracts.

Furthermore, natural extracts, extracted with organic solvents, of rumex crispus, saphora flavescens, nuphar japonicum, orange, salvia officinalis, achillea alpina, malva sylvestris, swertia japonica, hondoensis, thymus serpyllum L., augelica acutiloba, citrus aurantium L., birth extracts, equisetum arvens, luffa cylindrica, aesculus hippocastanum, saxifrage stolonifera meerburg, arnica montana, lily, artemisia princeps, paeonia lactiflora, aloe, gardenia jasminoides, chamaecyparis pisifera; neutralizing agents; antioxidants; dyes; fragrants; purified water; etc. may be formulated into the present composition.

As mentioned above, the external application composition of the present invention is sufficiently stable without using a surfactant, and therefore, a surfactant is not especially necessary to be added to the external application composition for the purpose of improving the external application composition. Please note that we do not intend to prevent the addition of a surfactant, and especially a surfactant may be formulated into the composition for the other purpose.

In the present external application composition, a surfactant may be formulated thereinto for the purpose of optionally improving the stability or for the other purpose.

Such surfactants are not specifically limited. Examples of the surfactants are nonionic surfactants such as sorbitan monolaurate, sorbitan monopalmitate, sorbitan sesquioleate, sorbitan trioleate, polyoxyethylene sorbitan monolaurate, polyoxyethyleneglycol monooleate, polyoxyethylene alkyl ether, polyethyleneglycol difatty acid ester, lauroyldiethanol amide, fatty acid dipropanol amide, maltitol hydroxy fatty acid ether, alkylated polysaccharide, alkylglucoside, sugar ester, polyether-modified silicone, etc.; cationic surfactants such as stearyl trimethylammonium chloride, benzalkonium chloride, laurylamine oxide, etc.; anionic surfactants such as sodium palmitate, sodium laurate, sodium laurylsulfate, potassium laurylsulfate, triethanolamine alkylsulfate, acylmethyl taurin salt, etc.; ampholytic surfactants; etc. These surfactants may be appropriately selected to formulate into the composition.

The type of the shape or form, in which the present external application composition may be adopted, is not specifically limited and all of the external application shapes or forms such as aqueous solution type, solubilized system, emulsified system, powder dispersion system, water-oil two-layer system, water-oil-powder three-layer system, etc. may also be possible to apply to the present invention.

The present external application composition may further contain, in addition to the above-mentioned essential constituents, water, alcohols, powders, preservatives, humectants, oils, buffer agents, chelating agents, coloring agents, fragrants, antioxidants, UV absorbers, other thickening agents, anti-aging agents, whitening agents, skin roughening improuers, etc.

### EXAMPLES

The present invention will now be further illustrated by, but is by no means limited to, the following Examples. All the amounts in the Examples are in terms of % by weight, unless otherwise noted.

Before the disclosure of the Formulation Examples, the evaluation methods used with respect to the present external application compositions are shown below.

### 1. Organoleptic Test of Feeling at Use

Each sample was evaluated with respect to the feelings at use by a panel consisting of 60 member (i.e., 30 men and 30 women), under the following criteria.
++: 40 or more member evaluated good
+: 20 to lees than 40 member evaluated good
±: 10 to less than 20 member evaluated good
-: only less than 10 member evaluated good

### 2. Viscosity Determination (Salt Resistance) Test

The viscosities of the external application compositions of Examples 1 - 3 and Comparative Examples 1 - 3 shown below are determined, after one hour from the preparation thereof, by a B-type viscometer (Rotor No. 3, 6 rpm) at a temperature of 25°C when sodium chloride is added and is not added.

The sodium chloride was added in an amount of 1.0% by weight, based upon the total weight of the composition, after the carboxyvinyl polymer and/or the alkyl-modified carboxyvinyl polymer were dissolved in a purified water, followed by neutralizing with potassium hydroxide.

### Example 1: Milky Lotion

| | Component | Amount (wt%) |
|---|---|---|
| (1) | 1,3-Butylene glycol | 5.0 |
| (2) | Carboxyvinyl polymer (Hivis Wako 104) | 0.1 |
| (3) | Gellan gum | 0.2 |
| (4) | Glycerol monostearic ester | 0.5 |
| (5) | Polyoxyethylene (10) monooleic ester | 0.5 |
| (6) | Liquid paraffin | 5.0 |
| (7) | Potassium hydroxide | 0.1 |
| (8) | Preservative | 0.1 |
| (9) | Chelating agent | q.s. |
| (10) | Antioxidant | q.s. |
| (11) | Fragrant | q.s. |
| (12) | Purified water | balance |

### Example 2: Milky Lotion

| | Component | Amount (wt%) |
|---|---|---|
| (1) | 1,3-Butylene glycol | 5.0 |
| (2) | Alkyl-modified carboxyvinyl polymer (PEMULEN TR-1) | 0.2 |
| (3) | Gellan gum | 0.2 |
| (4) | Liquid paraffin | 5.0 |
| (5) | Potassium hydroxide | 0.1 |
| (6) | Preservative | 0.1 |
| (7) | Chelating agent | q.s. |
| (8) | Antioxidant | q.s. |
| (9) | Fragrant | q.s. |
| (10) | Purified water | balance |

### Example 3: Milky Lotion

| | Component | Amount (wt%) |
|---|---|---|
| (1) | 1,3-Butylene glycol | 5.0 |
| (2) | Carboxyvinyl polymer (Hivis Wako 104) | 0.1 |
| (3) | Gellan gum | 0.2 |
| (4) | Alkyl-modified carboxyvinyl polymer (PEMULEN TR-1) | 0.2 |
| (5) | Liquid paraffin | 5.0 |
| (6) | Potassium hydroxide | 0.1 |
| (7) | Preservative | 0.1 |
| (8) | Chelating agent | q.s. |
| (9) | Antioxidant | q.s. |
| (10) | Fragrant | q.s. |
| (11) | Purified water | balance |

### Comparative Example 1: Milky Lotion

| | Component | Amount (wt%) |
|---|---|---|
| (1) | 1,3-Butylene glycol | 5.0 |
| (2) | Carboxyvinyl polymer (Hivis Wako 104) | 0.1 |
| (3) | Glycerol monostearate | 0.5 |
| (4) | Polyoxyethylene (10) monooleate | 0.5 |
| (5) | Liquid paraffin | 5.0 |
| (6) | Potassium hydroxide | 0.1 |
| (7) | Preservative | 0.1 |
| (8) | Chelating agent | q.s. |
| (9) | Antioxidant | q.s. |
| (10) | Fragrant | q.s. |
| (11) | Purified water | balance |

### Comparative Example 2: Milky Lotion

| | Component | Amount (wt%) |
|---|---|---|
| (1) | 1,3-Butylene glycol | 5.0 |
| (2) | Gellan gum | 0.2 |
| (3) | Glycerol monostearate | 0.5 |
| (4) | Polyoxyethylene (10) monooleate | 0.5 |
| (5) | Liquid paraffin | 5.0 |
| (6) | Potassium hydroxide | 0.1 |
| (7) | Preservative | 0.1 |
| (8) | Chelating agent | q.s. |
| (9) | Antioxidant | q.s. |
| (10) | Fragrant | q.s. |
| (11) | Purified water | balance |

### Comparative Example 3: Milky Lotion

| | Component | Amount (wt%) |
|---|---|---|
| (1) | 1,3-Butylene glycol | 5.0 |
| (2) | Alkyl-modified carboxyvinyl polymer (PEMULEN TR-1) | 0.2 |
| (3) | Liquid paraffin | 5.0 |
| (4) | Potassium hydroxide | 0.1 |
| (5) | Preservative | 0.1 |
| (6) | Chelating agent | q.s. |
| (7) | Antioxidant | q.s. |
| (8) | Fragrant | q.s. |
| (9) | Purified water | balance |

### Preparation Method

The test samples of Examples 1 - 3 and Comparative Examples 1 - 3 were prepared as follows.

To the purified water, the humectant, chelating agent, gellan gum (Note: only when the gellan gum is contained) were added and heated at 80°C to prepare an aqueous phase. On the other hand, the liquid paraffin, preservative, fragrant, etc. were added and mixed upon heating at 80°C. This was added to the aqueous those prepared above, followed by pre-emulsification. Further, to the preemulsified product, the carboxyvinyl polymer or alkyl-modified carboxyvinyl polymer were added, followed by stirring. The emulsified particles in the emulsion were made uniform by a homomixer, followed by deaeration, filtration, and cooling to obtain the test samples.

The milky lotions of each Example and each Comparative Example thus prepared were subjected to the above-mentioned organoleptic test for the application feelings at use and the viscosity measurement (Salt resistance). The results are as shown in Table 1 (i.e., Organoleptic test for application feelings at use) and Table 2 (i.e., Salt resistance test).

**Table 1**

| Item | Example | | | Comparative Example | | |
|---|---|---|---|---|---|---|
| | 1 | 2 | 3 | 1 | 2 | 3 |
| Feeling at use | ++ | ++ | ++ | ± | + | - |

As is clear from this result, the present external application composition containing the carboxyvinyl polymer and/or the alkyl-modified carboxyvinyl polymer in combination with the gellan gum provides the extremely improved application feelings at use, when compared with the Comparative Examples where the carboxyvinyl polymer and/or alkyl-modified carboxyvinyl polymer are used alone in the formulation.

**Table 2**

| | Example | | | Comparative Example | | |
|---|---|---|---|---|---|---|
| | 1 | 2 | 3 | 1 | 2 | 3 |
| NaCl not added | 5210 | 4750 | 5300 | 3420 | 1500 | 2580 |
| NaCl added | 5500 | 4860 | 5900 | 150 | 2100 | 120 |

As is clear from this result, the present external application composition containing the carboxyvinyl polymer and/or the alkyl-modified carboxyvinyl polymer and the gellan gum was observed that the viscosity thereof was increased due to the formulation together with the gellan gum. It was confirmed that this phenomenon was not affected by the presence or absence of a salt and the present external application composition was excellent also in the salt resistance.

The various types of formulation of the present external application compositions will now be explained. In the present external application compositions of these Examples, the above-mentioned two tests were carried out. As a result, the application feelings at use and the salt resistance are significantly excellent in the application feelings at use and the salt resistance when compared with the Comparative Examples in which only the gellan gum was not formulated thereon.

### Example 4: Cream

| | Component | Amount (wt%) |
|---|---|---|
| (1) | 1,3-Butylene glycol | 8.0 |
| (2) | Carboxyvinyl polymer (Mixture of Hivis Wako 104 and 105) | 0.1 |
| (3) | Gellan gum | 0.1 |
| (4) | Polyoxyethylene (10) monooleate | 1.0 |
| (5) | Glycerol monostearate | 1.0 |
| (6) | Cetyl alcohol | 1.0 |
| (7) | Bees wax | 0.5 |
| (8) | Vaseline | 2.0 |
| (9) | Squalane | 6.0 |
| (10) | Dimethyl polysiloxane | 2.0 |
| (11) | Potassium hydroxide | 0.1 |
| (12) | Preservative | q.s. |
| (13) | Coloring agent | q.s. |
| (14) | Chelating agent | q.s. |
| (15) | Fragrant | q.s. |
| (16) | Purified water | balance |

### Preparation Method

To the purified water, the preservative, coloring agent, the gellan gum were added and heated at 80°C to prepare the aqueous phase. On the other hand, to the oil components, the surfactant, preservative were added and heated at 70°C to prepare the oil component, which was added to the aqueous phase to effect the pre-emulsified product. To this pre-emulsified product, the carboxyvinyl polymer was added and the emulsified particles in the emulsion were made uniform by a homomixer, followed by deaeration, filtration, and cooling to obtain the desired cream.

### Example 5: Cream

| | Component | Amount (wt%) |
|---|---|---|
| (Aqueous phase) | | |
| (1) | 1,3-Butylene glycol | 6.0 |
| (2) | Polyethylene glycol 1500 | 4.0 |
| (3) | Gellan gum | 0.4 |
| (4) | Dimethylsiloxane·methyl (polyoxyethylene)siloxane copolymer | 1.0 |
| (5) | Alkyl-modified carboxyvinyl polymer (PEMULEN TR-1) | 0.1 |
| (6) | Purified water | balance |

| (Oil phase) | | |
|---|---|---|
| (7) | Stearyl alcohol | 3.0 |
| (8) | Hydrogenated lanolin | 3.0 |
| (9) | Squalane | 5.0 |
| (10) | Octyldodecanol | 8.0 |
| (11) | Preservative | q.s. |
| (12) | Antioxidant | q.s. |
| (13) | Fragrant | q.s. |

### Preparation Method

To the purified water, the preservative, gellan gum and the alkyl-modified carboxyvinyl polymer were added, followed by heating at 80°C to prepare the aqueous phase. On the other hand, the oil components were dissolved upon heating, the preservative, antioxidants and fragrant were added thereto and heated to at 80°C to prepare the oil phase. The oil phase was added to the previous aqueous phase and the emulsified particles were made uniform by a homomixer, followed by deaeration, filtration, cooling to obtain the desired cream.

### Example 6: Cosmetic Water

| | Component | Amount (wt%) |
|---|---|---|
| (1) | Dipropylene glycol | 10.0 |
| (2) | Polyethylene glycol 1500 | 5.0 |
| (3) | Alkyl-modified carboxyvinyl polymer (CARBOPOL 1342) | 0.1 |
| (4) | Gellan gum | 0.05 |
| (5) | Polyoxyethylene (20) oleyl alcohol ether | 0.5 |
| (6) | Ethanol | 5.0 |
| (7) | Potassium hydroxide | 0.1 |
| (8) | Fragrant | q.s. |
| (9) | Coloring agent | q.s. |
| (10) | Preservative | q.s. |
| (11) | Chelating agent | q.s. |
| (12) | Anti-fading agent | q.s. |
| (13) | Purified water | balance |

### Preparation Method

In a part of the purified water, the chelating agent was dissolved, followed by mixing with stirring the alkyl-modified carboxyvinyl polymer and the gellan gum. To the remaining part of the purified water, the preservative, anti-fading agent, etc. were added to be dissolved at a room temperature followed by adding the above solution to obtain a uniform aqueous solution. On the other hand, to the ethanol, the preservative, surfactant and fragrant to form an alcoholic solution, to which the above aqueous solution was added and mixed to be solubilized. Then, the coloring agent was added to adjust the color, followed by filtration to form the desired cosmetic water.

### Example 7: Pack

| Component | | Amount (wt%) |
|---|---|---|
| (1) | Polyethylene glycol 1500 | 5.0 |
| (2) | Dipropylene glycol | 5.0 |
| (3) | Sorbitol | 5.0 |
| (4) | Carboxyvinyl polymer (Hivis Wako 103) | 1.0 |
| (5) | Gellan gum | 1.0 |
| (6) | Polyoxyethylene (9) lauryl alcohol ether | 1.0 |
| (7) | Ethanol | 5.0 |
| (8) | Potassium hydroxide | 0.5 |
| (9) | Fragrant | q.s. |
| (10) | Preservative | q.s. |
| (11) | Purified water | balance |

### Preparation Method

To a part of the purified water, the carboxyvinyl polymer and the gellan gum were added, followed by stirring to be dissolved. On the other hand, the fragrant, preservative, surfactant were added to the ethanol and dissolved therein. This was added to the above aqueous phase to be solubilized. Finally, in the remaining part of the purified water, potassium hydroxide was dissolved, followed by adding thereto the above solubilized product, neutralizing, deaeration and filtration, to obtain the desired pack.

### Example 8: Sun Screening Agent

| | Component | Amount (wt%) |
|---|---|---|
| (Aqueous phase) | | |
| (1) | Dipropylene glycol | 6.0 |
| (2) | Carboxyvinyl polymer (CARBOPOL ETD2020) | 0.1 |
| (3) | Gellan gum | 0.5 |
| (4) | Potassium hydroxide | 0.1 |
| (5) | Purified water | balance |

| (Oil phase) | | |
|---|---|---|
| (6) | Octyl p-methoxycinnamate | 6.0 |
| (7) | Glyceryloctyl di p-methoxycinnamate | 2.0 |
| (8) | 4-tert-butyl-4'-methoxydibenzoyl methane | 2.0 |
| (9) | Oxybenzone | 3.0 |
| (10) | Oleyl oleate | 5.0 |
| (11) | Dimethyl polysiloxane | 3.0 |
| (12) | Vaseline | 0.5 |
| (13) | Cetyl alcohol | 1.0 |
| (14) | Sorbitan sesquioleate | 0.8 |
| (15) | Polyoxyethylene (20) oleyl alcohol ether | 1.2 |
| (16) | Antioxidant | q.s. |
| (17) | Preservative | q.s. |
| (18) | Fragrant | q.s. |

### Preparation Method

The oil phase and the aqueous phase were separately heated at 80°C to be dissolved. Then, the oil phase was added to the aqueous phase and emulsified using a homogenizer. The resultant emulsified product was cooled using a heat exchanger to obtain the desired sun screen agent.

### Example 9: Beauty Liquid

| | Component | Amount (wt%) |
|---|---|---|
| (1) | Ion-exchanged water | balance |
| (2) | Ethanol | 5 |
| (3) | Gellan gum | 0.3 |
| (4) | Xanthan gum | 0.1 |
| (5) | Aluminum magnesium silicate | 0.5 |
| (6) | 1,3-Butylene glycol | 5 |
| (7) | Dynamite glycerol | 3 |
| (8) | Squalane | 0.3 |
| (9) | Glyceryl tri 2-ethylhexanoate | 0.1 |
| (10) | Polyoxyethylene hydrogenated castor oil | 0.8 |
| (11) | Polyglyceryl diisostearate | 0.4 |
| (12) | Citric acid | 0.02 |
| (13) | Sodium citrate | 0.13 |
| (14) | Chelating agent | q.s. |
| (15) | Coloring agent | q.s. |
| (16) | Fragrant | q.s. |
| (17) | Preservative | q.s. |

### Preparation Method

The desired beauty liquid was prepared in the similar manner as in Example 6.

### Example 10: Milky Lotion

| | Component | Amount (wt%) |
|---|---|---|
| (1) | Ion-exchanged water | balance |
| (2) | Ethanol | 5 |
| (3) | Gellan gum | 0.2 |
| (4) | Alkyl-modified carboxyvinyl polymer | 0.1 |
| (5) | Xanthan gum | 0.05 |
| (6) | Aluminum magnesium silicate | 0.5 |
| (7) | 1,3-Butylene glycol | 5 |
| (8) | Dynamite glycerol | 5 |
| (9) | Decamethyl cyclopolysiloxane | 5 |
| (10) | Dimethyl polysiloxane | 5 |
| (11) | Liquid paraffin | 2 |
| (12) | Potassium hydroxide | 0.1 |
| (13) | Citric acid | 0.01 |
| (14) | Sodium citrate | 0.09 |
| (15) | Chelating agent | q.s. |
| (16) | Coloring agent | q.s. |
| (17) | Fragrant | q.s. |
| (18) | Preservative | q.s. |

### Preparation Method

The desired milky lotion was prepared in the similar manner as in Example 6.

### Example 11: Milky Lotion

| | Component | Amount (wt%) |
|---|---|---|
| (1) | Ion-exchanged water | balance |
| (2) | Ethanol | 0.2 |
| (3) | Gellan gum | 0.05 |
| (4) | Xanthan gum | 0.5 |
| (5) | 1,3-Butylene glycol | 5 |
| (6) | Dynamite glycerol | 5 |
| (7) | Squalane | 0.5 |
| (8) | Pentaerythritol tetra 2-ethylhexanoate | 0.5 |
| (9) | Dimethyl polysiloxane | 2 |
| (10) | Vaseline | 0.5 |
| (11) | Polyoxyethylene hydrogenated castor oil | 0.3 |
| (12) | Citric acid | 0.01 |
| (13) | Sodium citrate | 0.09 |
| (14) | Chelating agent | q.s. |
| (15) | Coloring agent | q.s. |
| (16) | Fragrant | q.s. |
| (17) | Preservative | q.s. |

### Preparation Method

The desired milky lotion was prepared in the similar manner as in Example 6.

As explained above, according to the present invention, the external application composition having remarkably improved application feelings at use and having excellent salt resistance as advantageously provided.

## Claims

1. An external application composition comprising:
(A) at least one carboxyvinyl polymer selected from the group consisting of carboxyvinyl polymers and alkyl-modified carboxyvinyl polymers; and
(B) gellan gum.

2. An external application composition as claimed in claim 1, wherein the content of the component (A) is 0.01 - 3% by weight, based upon the total amount of the composition.

3. An external application composition as claimed in claim 1 or 2, wherein the content of the component (B) is 0.01 - 1.5% by weight, based upon the total amount of the composition.

4. An external application composition as claimed in any one of claims 1 - 3, wherein the weight ratio of the component (B) to the component (A) is 1:100 to 150:1.

5. An external application composition as claimed in any one of claims 1 - 4, wherein a surfactant is not contained therein for the purpose of improving the stability of the external application composition.
